# EUROPEAN PATENT APPLICATION

(11) **EP 1 666 560 A1**
(43) Date of publication of application: **07.06.2006**
(21) Application number: 04773065.0
(22) Date of filing: 08.09.2004
(51) Int. Cl.: C09K 11/06, C01B 33/12, G01N 33/543, G01N 33/533, G01N 33/566

(54) **NOVEL FINE FLUORESCENT PARTICLE**

(30) Priority: 08.09.2003 JP 2003316006
(71) Applicant: WASEDA UNIVERSITY, Tokyo 169-0071 (JP)
(72) Inventor: MATSUMOTO, Kazuko, Setagaya-ku, Tokyo 1550032 (JP); NISHIOKA, Takuya, Shinjuku-ku, Tokyo 1690075 (JP)
(74) Representative: Albrecht, Thomas
(86) International application number: PCT/JP2004/013392
(87) International publication number: WO 2005/023961

(57) **Abstract**

Novel silica particles having bondable functional groups on the surface thereof and containing a fluorescent rare-earth complex **characterized by** being uninfluenced by the surrounding environment including a buffer and a substance to be labeled. The silica particles containing a fluorescent rare-earth complex specifically are ones which comprise silica particles containing a fluorescent rare-earth complex selectively in an inner layer thereof and the surface of which consists substantially of silica. Also provided are: a fluorescent labeling agent comprising the silica particles; a method of fluorescent labeling which comprises using the silica particles as a labeling agent; and a method for fluorescent determination and a reagent for fluorescent determination which employ the fluorescent labeling agent.

## Description

### TECHNICAL FIELD

The present invention relates to silica particles containing a fluorescent rare-earth complex, in more detail, silica particles containing a fluorescent rare-earth complex, which selectively contains a fluorescent rare-earth complex substantially in an inner layer thereof, in further more detail, silica particles containing a fluorescent rare-earth complex, which selectively contains a fluorescent rare-earth complex substantially in an inner layer thereof and the surface thereof substantially consists of silica; a fluorescent labeling agent comprising said silica particles; and a method of fluorescent labeling which comprises using said silica particles as a labeling agent; along with a method of fluorimetry and a fluorimetry reagent using said fluorescent labeling agent.

### BACKGROUND ART

Conventionally, immunoassay utilizing an antigen-antibody reaction or DNA hybridization has been widely used as an analysis method for microanalysis in biological samples. In these analysis methods, it is required to use a labeling agent to label an antibody or DNA and the like, and as a labeling agent enabling high-sensitive detection, a labeling agent by means of fluorescence, a labeling agent by means of an radioisotope, a labeling agent by means of an enzyme, and the like have been widely used.

Labeling with a radioisotope provides high sensitivity, however, it has a disadvantage of involving risks in storage, use and treatment. In addition, labeling with an enzyme has problems that since molecular weight of the enzyme is large, it is easily influenced by the surrounding factors such as temperature, and thus unstable and inferior in reproducibility, and further, by binding the enzyme labeling agent to a substance to be labeled, the activity of the enzyme and a substance to be labeled decreases.

In addition, as a labeling method with a fluorescence, labeling with an organic fluorescent dye (for example, fluorescein, rhodamine and dansyl chloride) has been known, however, it has a disadvantage that highly sensitive measurement is difficult due to the inhibition of fluorescent detection from the labeling agent caused by background noise derived from scattering of excited light or background noise derived from fluorescence of other coexisting substances in a sample.

As a labeling method by means of fluorescence besides this method, labeling with a fluorescent rare-earth complex is known. A fluorescent rare-earth complex has characteristics of long fluorescent lifetime (a fluorescent rare-earth complex has a fluorescent lifetime of several tens to not shorter than several hundreds micro seconds, compared with a fluorescence lifetime of several nano seconds in usual organic fluorescent substances), large Stokes shift and sharp fluorescence emission peak. These characteristics are exploited to time-resolved fluorometry whereby removing short lifetime background noise derived from excitation light or coexisting substances and enabling highly sensitive measurement. A time-resolved fluorometry method has already been developed by using a fluorescent rare-earth complex as a labeling agent based on such characteristics.

The present inventors have already developed many labeling agents using a fluorescent rare-earth complex, and have examined on their application to a time-resolved fluorometry method. However, synthesis of a complex having a bonding group to a substance to be labeled has many reaction steps and involves difficulty, and a synthesized complex may sometimes change largely depending on property of a bonding group. In addition, it has a disadvantage that kind of a buffer solution which can be used is limited when chelating strength of a complex is insufficient. Furthermore, when bonding site of a substance to be labeled are scarce, number of molecules of a labeling agent of a fluorescent rare-earth complex per molecule of a substance to be labeled is limited, thereby resulting in limitation of sensitivity improvement.

On the other hand, a DNA probe method has widely been used in DNA analysis. In this method, a standard sample and a test sample are labeled using two or more kinds of labeling agents, and they are furnished to the same probe DNA, and thus DNA amount in a test sample is quantitatively determined based on the results of competitive hybridization. However, this method requires introduction of a labeling agent to a test sample, and this operation results in a possibility of changing abundance ratio of target sequence in a test sample. Moreover, introduction of a labeling agent may lower double-strand formation ability of DNA, and may provide wrong result in diagnosis of gene mutation or polymorphism.

In addition, not only rare-earth metals but also ions of metals such as calcium, zinc and manganese are used as fluorescence emission substance for an emission substance of three primary colors, such as for a plasma display panel (PDP). Because these emission substances are for panel applications, not only fluorescence emission intensity but also mechanical strength and persistency are required, and many efforts have been made. For example, the following examples have been reported: a fluorescence emission substance using the surface of silica particles having a particle diameter of 5 to 20 µm and a specific surface area of 100 to 800 m²/g, as a silicate salt by means of these fluorescent metal ions (see Patent Reference 1), a fluorescence emission substance including the fine zinc oxide particles in silica particles (see Patent Reference 2), a fluorescent emission substance produced by comprising an organic carboxylic acid in silica particles having a specific surface area of not larger than 100 m²/g, obtained by hydrolysis of alkoxysilane (see Patent Reference 3), ones carrying a fluorescent substance at the surface of an inorganic substance such as silica (see Patent Reference 4), ones obtained by coating the surface of emission substance particles with a silica film having a refractive index of not smaller than 1.435 (see Patent References 5 and 6), a fluorescent substance obtained by introducing a complex of rare-earth metal such as Eu or Tb by a sol-gel method, into organic-inorganic composite type matrix structure, wherein at least one part of 3D structure of silica is substituted with an organic substance (see Patent Reference 7), and the like.

In addition, silica particles themselves are widely used also as a carrier of DNA or protein and the like, for example, such a method is known for coating the surface of fine inorganic particles such as silica with a crosslinked resin for immobilizing DNA thereon (see Patent Reference 8). Furthermore, a method is reported for immobilization of a ligand of a complex for labeling on the surface of a solid supporting substance such as silica, for bonding metals thereto (see Patent Reference 9).

However, fluorescent substances described in Patent References 1 to 7 are ones with fluorescent substances distributed in whole area of a particle, and thus utilized as fluorescent substances for PDP and the like, aiming at improvement of lifetime or mechanical strength of fluorescent substances. Since these fluorescent substances are distributed also to the surface of the particles whereby easily influenced by the surrounding environment such as a buffer solution, and further reactive functional groups for carrying DNA or protein are scarce at the particle surface, they are not suitable for fluorescent particles for labeling.

As prior technical references relevant to the present invention, there are the following Patent References, which are incorporated in this specification by reference.
1.JP-A-2003-213254
2. JP-A-2003-201473
3.JP-A-2003-155478
4.JP-A-2002-180041
5. JP-A-2002-69442
6. JP-A-200 1-55567
7. JP-A-09-227861
8. JP-A-2001-183378
9.JP-A-09-505061

### DISCLOSURE OF INVENTION

The present invention has been proposed under the above circumstance and aims at providing novel silica particles internally containing many fluorescent rare-earth complexes and characterized in being uninfluenced by the surrounding environment including such as a buffer solution and substance to be labeled; a fluorescent labeling agent comprising said silica particles; and a method for fluorescent labeling using said silica particles as a labeling agent; along with a method for fluorescent determination using said fluorescent labeling agent, and the like.

### BRIEF DESCRIPTION OF THE DRAWINGS

Fig. 1 shows fluorescence spectrum of silica particles containing a BPTA-Tb complex of the present invention. Concentration of silica particles in a dispersion solution=0.1 mg/ml in water. Excitation wavelength=320 nm.
Fig. 2 shows investigation results of the effects on fluorescence intensity of silica particles containing a BPTA-Tb complex of the present invention (right side of Fig. 2), and a solution of a BPTA-Tb complex, in buffer solution at various pH values.
Fig. 3 shows fluorescence spectrum of silica particles containing a BTTA-Eu complex of the present invention. Concentration of silica particles in a dispersion solution=0.1 mg/ml in water. Excitation wavelength=340 nm.
Fig. 4 shows fluorescence intensity obtained by time-resolved fluorometry of DNA labeled by silica particles of the present invention.
Fig. 5 shows a scanning electron microscope photograph of silica particles containing a BTTA-Eu complex of the present invention. The particles are coated with platinum-palladium.
Fig. 6 shows fluorescence intensity obtained by time-resolved fluorometry of DNA labeled by silica particles without surface modification of the present invention.
Fig. 7 shows fluorescence intensity obtained by time-resolved fluorometry of DNA labeled by silica particles introduced with bivalent intercalators at the surface thereof of the present invention.
Fig. 8 shows fluorescence intensity obtained by time-resolved fluorometry of DNA labeled by silica particles introduced with monovalent intercalators at the surface thereof of the present invention.

### BEST MODE FOR CARRYING OUT THE INVENTION

The present inventors have comprehensively studied a way to solve the above-described problems, and found that silica particles, wherein a fluorescent substance is selectively present inside the silica particles, and a fluorescent substance composed of structure having a surface layer substantially comprising silica is included outside the inner structure can be obtained by a simple and convenient method.

That is, the present invention relates to silica particles containing a fluorescent rare-earth complex, in more detail, silica particles containing a fluorescent rare-earth complex which selectively contains a fluorescent rare-earth complex substantially in an inner layer thereof, in further more detail, the present invention relates to silica particles containing a fluorescent rare-earth complex which selectively contains a fluorescent rare-earth complex substantially in an inner layer thereof and the surface thereof substantially consists of silica. In addition, the present invention relates to a method for producing silica particles containing a fluorescent rare-earth complex by emulsion polymerization of tetraalkoxy orthosilicate in the presence of a fluorescent rare-earth complex.

Further, the present invention relates to a fluorescent labeling agent comprising the above-described silica particles containing a fluorescent rare-earth complex of the present invention; a fluorescence labeled nucleic acid probe wherein a nucleic acid probe is bonded at the surface of said silica particles containing a fluorescent rare-earth complex; a method for detection of double-stranded DNA using silica particles containing a fluorescent rare-earth complex and introduced with intercalators to double-stranded DNA onto said silica particles containing a fluorescent rare-earth complex; and a kit for target molecule determination comprising the above-described fluorescent labeling agent of the present invention, markers containing said fluorescent labeling agent, and material for target molecule determination.

Silica particles containing a fluorescent rare-earth complex of the present invention are different from silica particles having fluorescent substances nearly uniformly compounded in every part of silica particles such as a fluorescence emission substance produced by a conventional sol-gel method, and they selectively incorporate many fluorescent rare-earth complexes inside silica particles and thus silica substantially remain intact at the vicinity of the particle surface. Therefore, silica particles containing a fluorescent rare-earth complex of the present invention are ones wherein a fluorescent complex inside is uninfluenced by the surrounding environment, and in addition, an activated substituent bondable to a substance to be labeled can easily be introduced at the particle surface, and furthermore, double-stranded DNA can directly be labeled by introducing intercalator molecules to the particle surface.

Silica particles containing a fluorescent rare-earth complex of the present invention have features in having two-layer structure, that is, an inner layer substantially containing a fluorescent rare-earth complex, and a silica surface layer substantially comprised of silica and substantially not containing a fluorescent rare-earth complex. A fluorescent rare-earth complex may be present at any part of a particle, such as the inner part or the surface of a particle, however, moieties for a fluorescent rare-earth complex to fulfill function as a fluorescent complex are preferably at the inside of the silica particles. In the present invention, the case when a fluorescent rare-earth complex can fulfill function as a fluorescent complex in the inner layer of the silica particles is referred to as "a fluorescent rare-earth complex is substantially present in the inner layer of the silica particles".

Silica particles containing a fluorescent rare-earth complex of the present invention can be produced by various methods which can form the above-described two-layered structure, for example, a method for an emulsion polymerization of silica material such as tetraalkoxy orthosilicate in the presence of a fluorescent rare-earth complex is simple and convenient, and can efficiently form objective two-layered structure, and this method is included as a preferable method for production of silica particles containing a fluorescent rare-earth complex of the present invention.

For example, surfactant is added to a mixed stock of a water immiscible organic solvent and water, and further add silica material such as tetraalkoxy orthosilicate then emulsification by such as ultrasonic treatment is carried out.
An emulsion solution thus obtained is referred to as solution I. Subsequently, an emulsion solution containing a catalyst and a fluorescent rare-earth complex is similarly prepared as solution II. Then, solution I and solution II are mixed and subjected to emulsion polymerization to produce silica particles containing a fluorescent rare-earth complex of the present invention.

As the water immiscible organic solvent of solution I, any one can be used as long as it enables to form water and emulsion solution in the presence of a surfactant, however, one which is a solvent for silica material such as tetraalkoxy orthosilicate is further preferable. As an example of the water immiscible organic solvent of solution I, one kind of hydrocarbon-based solvents such as cyclohexane, hexane, octane, nujol and the like and one kind of aliphatic alcohols such as hexanol and heptanol and the like, or a mixture of two or more kinds thereof is included. As the surfactant for emulsification, any of a cation surfactant, an anion surfactant and a nonion surfactant may be used, however, a nonion surfactant such as Triton X and Brij is preferable. Use amount of the surfactant may be any amount as long as it is required for emulsification.

The solution I is prepared by emulsification by means of the addition of water into a mixture of the water immiscible organic solvent and the surfactant, and then by the addition of silica material such as tetraalkoxy orthosilicate thereto. As for silica material to be used, any one may be used as long as it enables to form silica particles by emulsion polymerization in the presence of a catalyst and not especially limited, however, tetraalkoxy orthosilicate is included as a preferable silica material. As an alkoxyl group of tetraalkoxy orthosilicate, a branched or straight chained lower alkoxyl group having about 1 to 8 carbon atoms, preferably about 1 to 5 carbon atoms is preferable. Four alkoxyl groups in tetraalkoxy orthosilicate are preferably the same, however, they may not necessarily be the same. As a preferable alkoxyl group, for example, a methoxy group, an ethoxy group, a n-propoxy group, an i-propoxy group, and the like are included, and specifically, tetraethoxy orthosilicate, tetramethoxy orthosilicate, and the like are included.

The solution II is an emulsion solution containing a fluorescent rare-earth complex and a catalyst, and an emulsion solution having a similar composition as in solution I is preferable. An emulsion solution can be produced by a similar method as in solution I, for example, it is preferable that an aqueous solution of a fluorescent rare-earth complex and a catalyst is mixed with a mixed solution of the water immiscible organic solvent shown in the above-described solution I and the surfactant, and then it is subjected to stirring or ultrasonic treatment and the like to emulsify. As the water immiscible organic solvent or the surfactant in this case, use of the same one as in the above-described solution I is preferable.

A preferable method for mixing solution I and solution II is by gradually adding solution II to solution I. According to this method, a fluorescent rare-earth complex and a catalyst are added gradually in portions into the emulsion solution of silica material, and because incorporation of a fluorescent rare-earth complex and polymerization of orthosilicate is carried out at the interface in excess of alkoxy orthosilicate, silica particles of the present invention can be obtained simply and in small particle diameter (nano order).

The catalyst used here is not especially limited as long as it enables to polymerize silica material by an acid or a base, however, catalyst which can stably co-exist with a fluorescent rare-earth complex is preferable. Specific catalysts thereof include inorganic bases such as ammonia and sodium hydroxide, and inorganic acids such as hydrochloric acid and sulfuric acid.

A fluorescent rare-earth complex of the present invention is not especially limited as long as it has a rare-earth metal as a center element, however, fluorescent rare-earth complex having characteristics of long fluorescent lifetime (a fluorescent rare-earth complex has a fluorescent lifetime from several tens to not shorter than several hundreds micro seconds, compared with a fluorescence lifetime of several nano seconds in usual organic fluorescent substances), large Stokes shift and a sharp fluorescence emission peak, and a time-resolved fluorometry that can be utilized as a labeling agent is preferable. Preferable fluorescent rare-earth complex includes a fluorescent rare-earth complex comprising ligands represented by the following formulae (1) to (6) 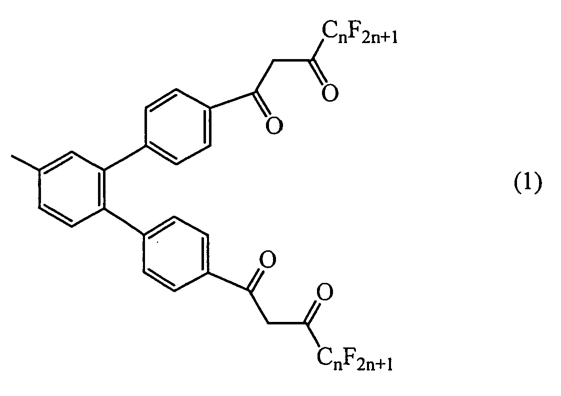 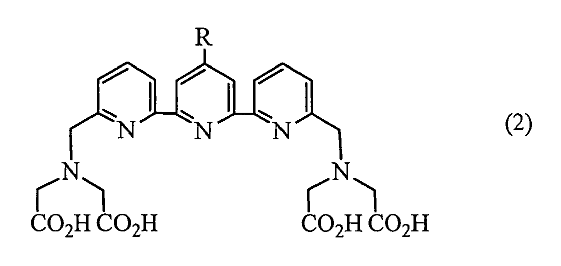 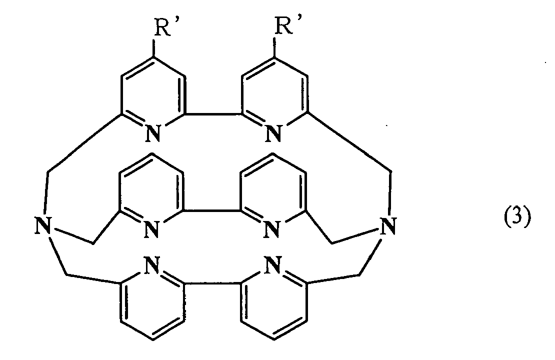 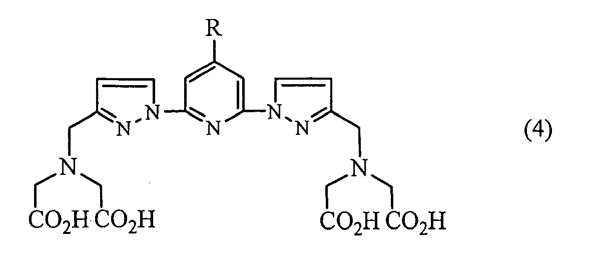 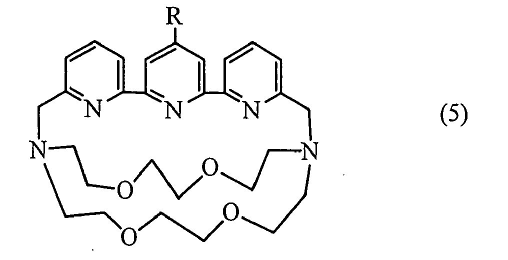 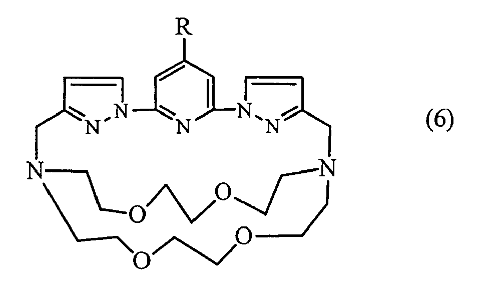 (In the formulae (1) to (6), n represents an integer of 1 to 4, R represents an optionally substituted aryl group, and R' represents a hydrogen atom, an amino group, a hydroxyl group, a carboxyl group, a sulfo group or an isothiocyanato group.), or such ligands having the following structures: 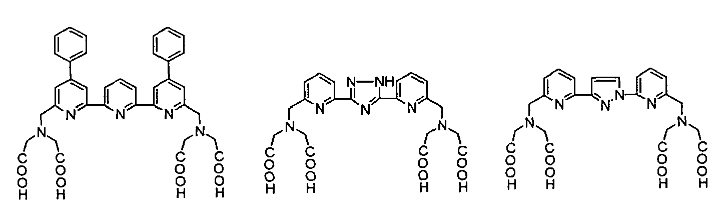 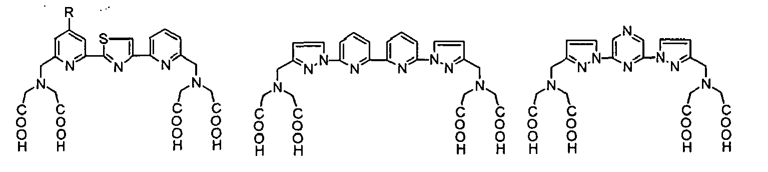 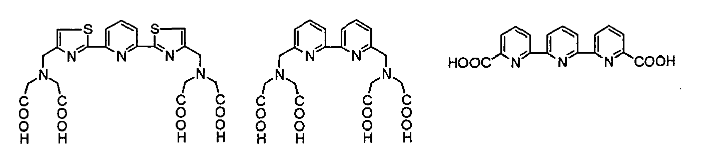 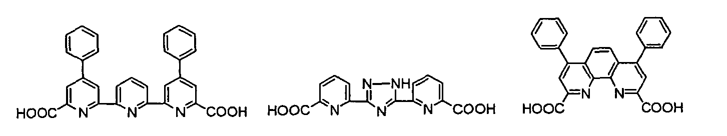 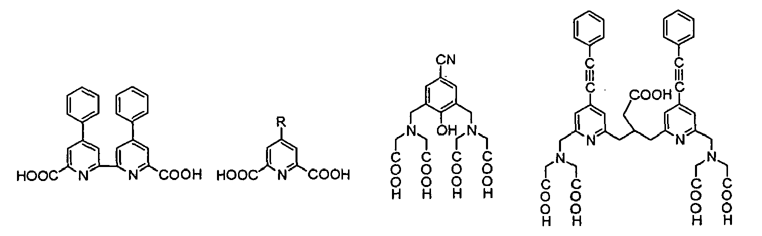 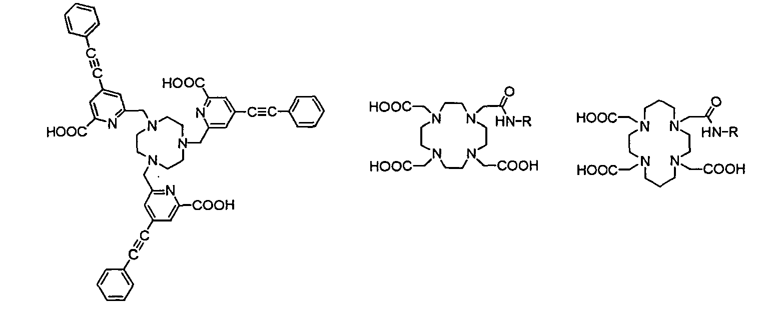 and a rare-earth element.

In addition, at least one kind of a compound selected from the group consisting of the compounds represented by the following formulae may also be used as a ligand:

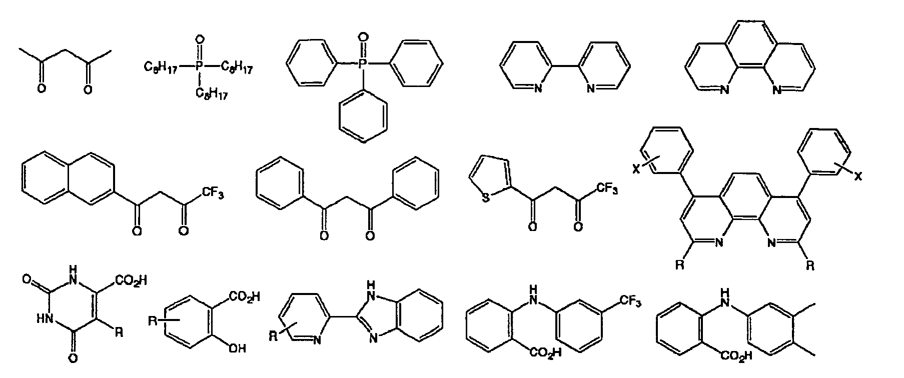

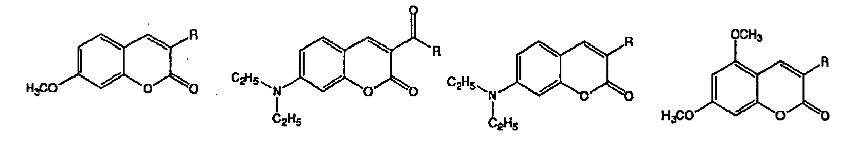

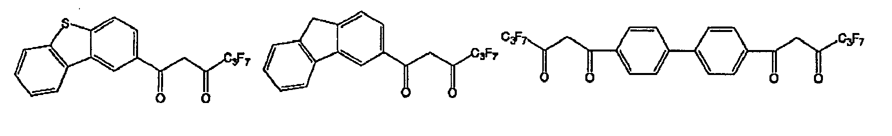

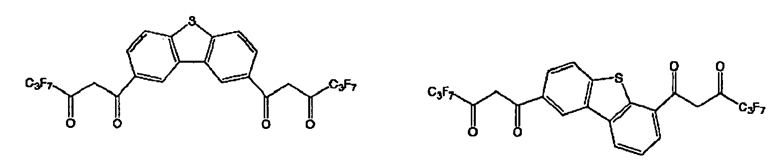

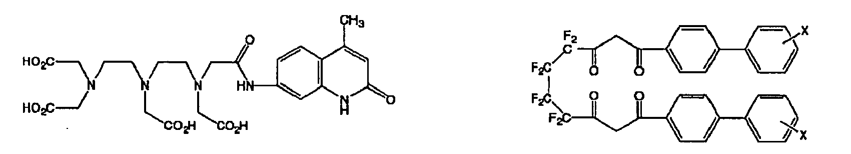

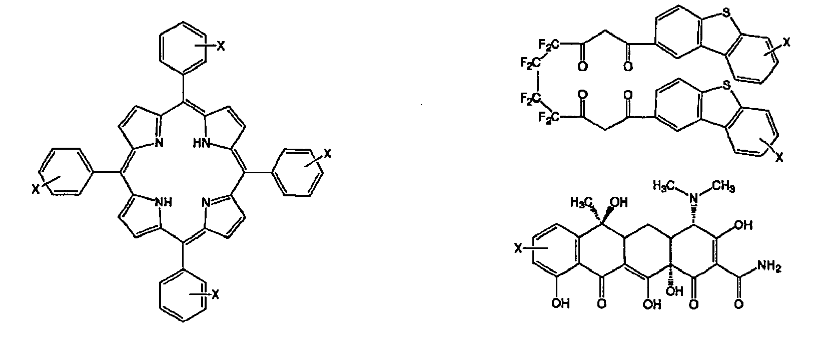

It is one of the characteristics of the present invention to use at least one kind of a compound selected from the group consisting of above-described compounds group as a ligand of a fluorescent rare-earth complex.

As a rare-earth metal of a fluorescent rare-earth complex of the present invention, terbium or europium, in more detail a terbium ion or a europium ion is included.

An aryl group in formulae (2), and (4) to (6) includes one or more than one 5- or 6-membered monocyclic, polycyclic or condensed-ring aromatic ring group, and said aromatic ring may have one or more hetero atoms comprising a nitrogen atom, an oxygen atom or a sulfur atom in the ring. As a preferable aryl group, for example, a phenyl group, a naphthyl group, a biphenyl group, a pyridyl group, an imidazolyl group, and the like are included. As a substituent for these aryl groups, a branched or straight chained lower alkyl group having 1 to 8 carbon atom(s), preferably about 1 to 5 carbon atom(s); a branched or straight chained lower alkoxyl group having 1 to 8 carbon atom(s), preferably about 1 to 5 carbon atom(s); a halogen atoms such as chlorine, fluorine and bromine; a hydroxyl group, an amino group, a carboxyl group, a sulfo group, and the like, are included. In addition, an R' group in formula (3) is a substituent to a pyridine ring, and when it is substituted, it may be optionally substituted with such as an amino group, a hydroxyl group, a carboxyl group, a sulfo group and an isothiocyanato group.

More specifically, for example, as a fluorescent rare-earth complex, a terbium-based fluorescent complex (hereinafter abbreviated as BPTA-Tb³⁺) comprising a terbium ion and an organic ligand N,N,N',N'-{2,6-bis(3'-aminomethyl-1'-pyrazolyl)-4-phenylpyridine}tetrakis(acetic acid) (one represented by the above-described formula (2), wherein a substituent R is a phenyl group and hereinafter abbreviated as BPTA), is included, and synthesis of BPTA and an N-hydroxy succinimide monoester thereof (hereinafter abbreviated as NHS-BPTA) can be carried out in accordance with a method reported by the present inventors (J. Yuan, G. Wang, K. Majima, K. Matsumoto; Anal. Chem., 2001, 73, 1869). Chemical structure of BPTA-Tb³⁺ is shown below:

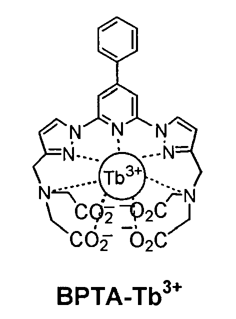

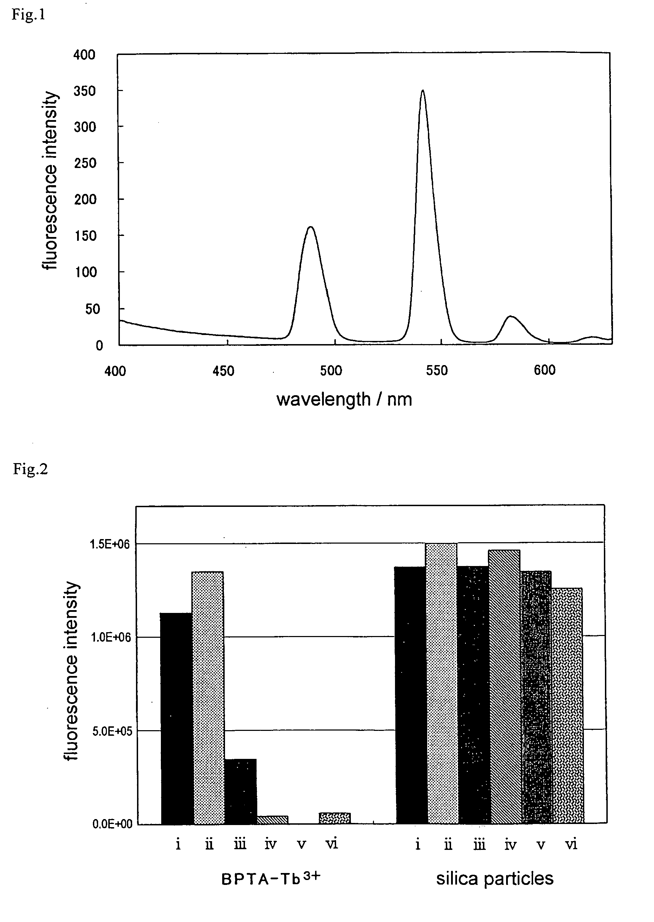

Investigation result on fluorescence characteristics of silica particles of the present invention containing BPTA-Tb³⁺, under the conditions of a concentration of a dispersion solution of silica particles of 0.1 mg/ml and an excitation wavelength of 320 nm is shown in Fig. 1. Abscissa axis of Fig. 1 represents wavelength (nm) and ordinate axis represents fluorescence intensity. From the result, silica particles of the present invention was found to show fluorescence spectrum nearly the same as in BPTA-Tb³⁺ introduced.

Then stability of silica particles of the present invention was examined. Silica particles of the present invention containing BPTA-Tb³⁺ was dispersed in buffer solutions having various pH values, such as a buffer solution of 15 mM Tris-hydrochloric acid (pH 7.4), a buffer solution of 15 mM Tris-hydrochloric acid and 50 mM sodium chloride (pH 7.4), buffer solutions of 1×SSC, 1×PBS, 1×TE, and 100 mM carbonic acid (pH 9.0), to prepare each 1.0 mg/l of a dispersion solution, which was subjected to time-resolved fluorometry. As a control, a BPTA-TB³⁺ solution in the same buffer solution was used. Results are shown in Fig. 2. Ordinate axis of Fig. 2 represents fluorescence intensity and the left side of abscissa axis represents the case of a BPTA-Tb³⁺ solution and the right side represents the case of silica particles of the present invention. Each of the six bar graphs represents, from the left side, as follows: (i) A buffer solution of 15 mM Tris-hydrochloric acid (pH 7.4) (black painted), (ii) a buffer solution of 15 mM Tris-hydrochloric acid and 50 mM sodium chloride (pH 7.4) (gray part shown by dotts), (iii) a 1×SSC buffer solution (dark gray), (iv) a 1×PBS buffer solution (downward oblique line), (v) a 1×TE buffer solution (ph 8.0) (a little pale gray) and (vi) a 100 mM carbonic acid buffer solution (pH 9.0) (wave line pattern). Determination conditions were as follows: Excitation wavelength=320 nm, determination wavelength=545 nm, delay time=0.5 msec., window time=1.4 msec. and cycling time=2.0 msec.

As a result, dramatic decrease in fluorescence intensity was observed in a BPTA-Tb³⁺ solution (left side of Fig. 2), in particular, in a 1×PBS buffer solution, a 1×TE buffer solution and a 100 mM carbonic acid buffer solution (ph 9.0). However, fluorescence of silica particles of the present invention showed nearly constant value, which suggested that a fluorescent rare-earth complex was present inside the particle whereby completely protected from the surrounding environment such as a buffer solution.

In addition, a fluorescent complex represented by the following formula (hereinafter abbreviated as BTTA-Eu³⁺); 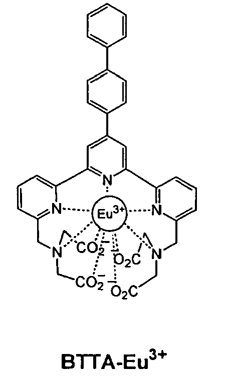 which comprises europium ion and 2,2',2",2"'-[4'-biphenyl-2,2':6',2"-terpyridine-6,6"-diyl]bis(methylenenitrilo) tetraacetic acid (hereinafter abbreviated as BTTA) represented by the following formula: 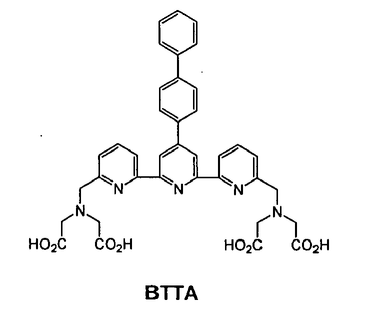 which corresponds to one represented by the above-described formula (2), wherein an R group is a biphenyl group, as organic ligands, is included.

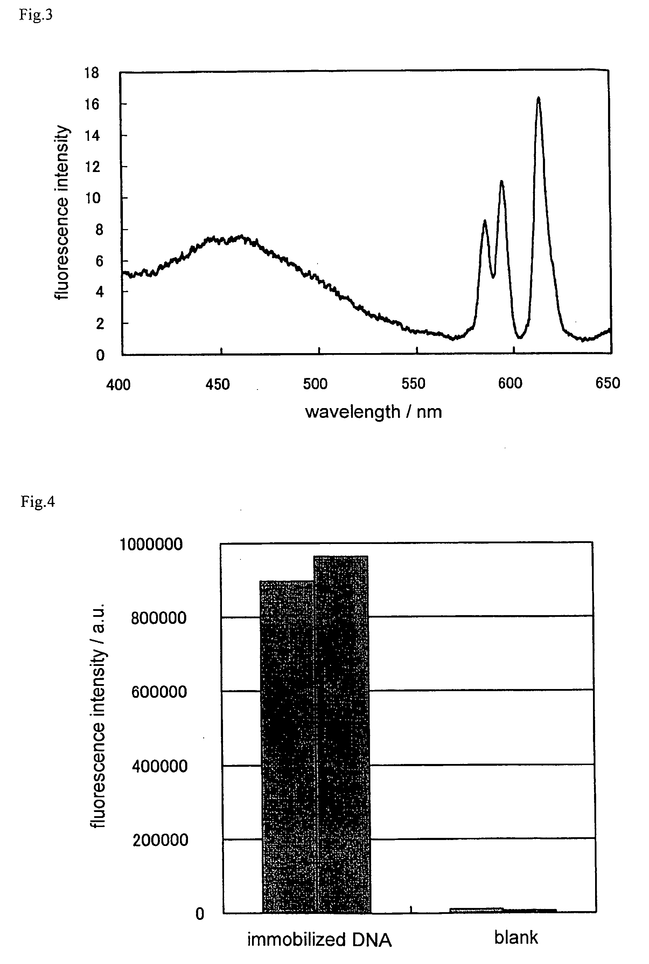

Investigation result of fluorescence characteristics of silica particles of the present invention containing BTTA-Eu³⁺, under the conditions of a concentration of a dispersion solution of silica particles of 0.1 mg/ml and an excitation wavelength of 340 nm, is shown in Fig. 3. Abscissa axis of Fig. 1 represents wavelength (nm) and ordinate axis represents fluorescence intensity. From the result, silica particles of the present invention were fond to show fluorescence spectrum nearly the same as in BTTA-Eu³⁺ introduced.

The surface of silica particles containing a fluorescent rare-earth complex of the present invention is substantially consisted of silica, and has features of protecting a inner fluorescent rare-earth complex from the surrounding environment, and at the same time has functional groups bondable to various compounds, at the surface thereof. Direct bonding of a substance to be labeled through these functional groups is also possible, however, it is also possible to bond by means of introducing bonding groups to a substance to be labeled, to functional groups at the surface of silica particles. As these bonding groups, various functional groups such as a cyano group, an alkylamino group, an alkanethiol group, an alkylcarboxyl group and an alkylaldehyde group are included. These functional groups having these alkyl groups are bonded to the surface of silica particles in such structure as Si-alkylamine or Si-alkanethiol, through a silicon atom and an alkyl group at the surface of silica particles.

Various molecules can be bonded at the surface of silica particles containing a fluorescent rare-earth complex of the present invention through the above-described bonding groups or directly. As these molecular species, ones enabling specific bond such as an antibody, an antigen, a receptor and a nucleic acid probe are included. For example, as a nucleic acid probe, a DNA or RNA molecule having a length of about 10 to 50 bases, 10 to 30 bases or 10 to 25 bases, is bonded to the surface of silica particles of the present invention in advance, and by means of hybridization with nucleic acid to be detected, the hybridized nucleic acid probe can be detected by a fluorescent label. Therefore, the present invention provides a fluorescence labeled nucleic acid probe comprising a nucleic acid probe such as DNA or RNA bonded at the surface of silica particles containing a fluorescent rare-earth complex of the present invention.

In addition, intercalator molecules can be introduced to the silica surface directly or through the above-described bonding groups. These intercalator molecules can specifically intercalate to double-stranded hybrid of nucleic acids, and thus enable to specifically label double-stranded DNA/DNA, RNA/RNA, PNA/PNA, DNA/RNA, DNA/PNA and PNA/RNA. For example, by hybridization of DNA to be detected and probe DNA, and by the addition of silica particles of the present invention or silica particles introduced with intercalators, easy detection of whether hybridization occurred or not becomes possible. Therefore, the present invention provides a method for detection of double-stranded hybrids of nucleic acids using silica particles containing a fluorescent rare-earth complex of the present invention, or silica particles containing a fluorescent rare-earth complex introduced with intercalators.

As these intercalator molecules, the following molecules can be exemplified:

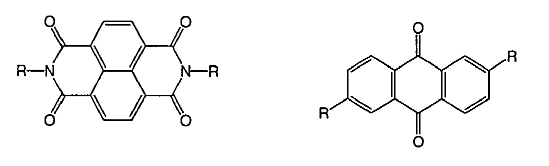

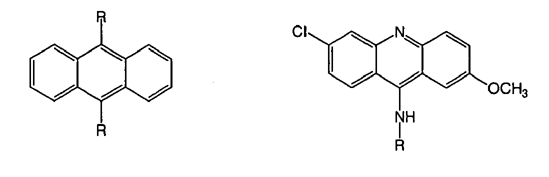

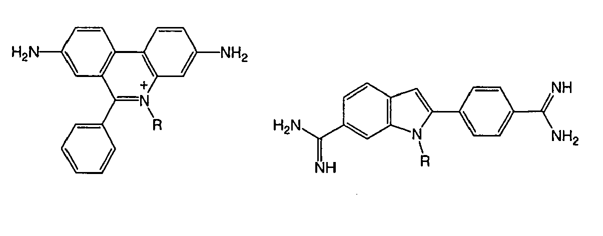

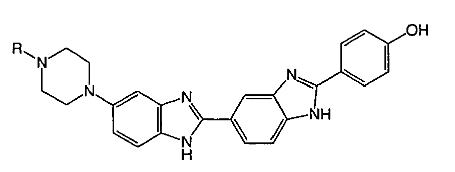

R groups in the above-mentioned formulae represent linker moieties to be bonded to silica particles. As these linkers, polyether, polyethylene polyamines, an alkylene group, and the like are included. In more detail, for example, an intercalator molecule having a linker represented by the following formula is included:

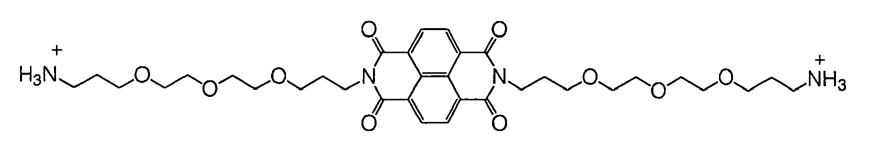

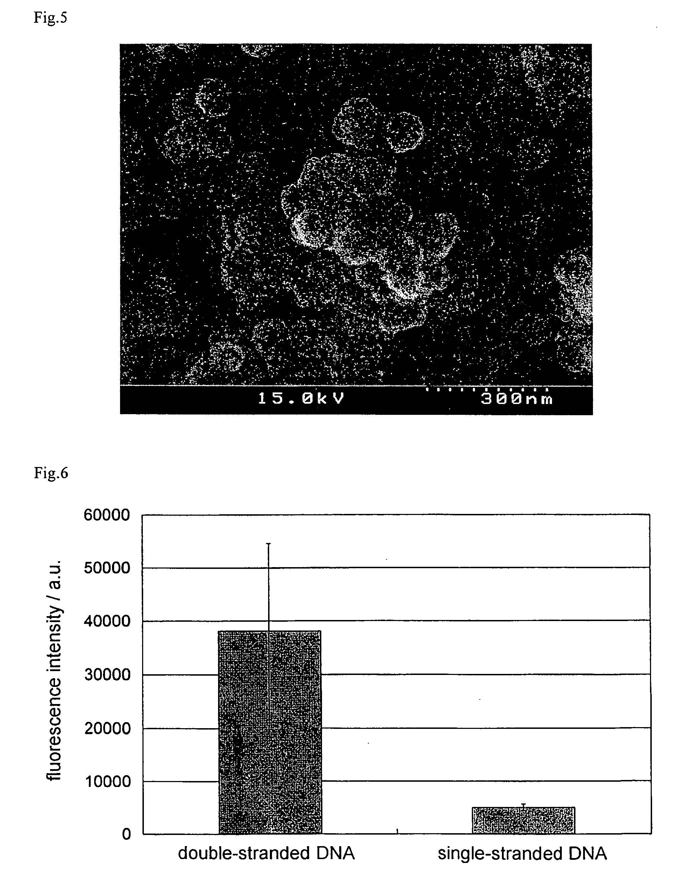

This intercalator molecule was immobilized at the surface of silica particles of the present invention containing BPTA-TB³⁺ by using a cyano group as a bonding group. 1 mg of silica particles were dispersed in a buffer solution, which was then added to a microtiter plate immobilized with double-stranded DNA to be subjected to time-resolved fluorometry. As a blank, the microtiter plate not immobilized with double-stranded DNA was used as a control. The results are shown in Fig. 4. Two bars at the left side of Fig. 4 show the case when the microtiter plate is immobilized with double-stranded DNA, while two bars at the right side show the case of the blank. Ordinate axis of Fig. 4 represents fluorescence intensity (a. u.). Determination conditions were as follows: Excitation wavelength=320 nm, determination wavelength=545 nm, delay time=0.5 msec., window time=1.4 msec. and cycling time=2.0 msec.

As a result, it was found that hybridized double-stranded DNA could be detected extremely clearly by means of a fluorescent label bonded with intercalator molecules at silica particles of the present invention.

As shown above, silica particles containing a fluorescent rare-earth complex of the present invention enables bonding groups or intercalator molecules to bond a substance (a labeled substance) to be labeled at the surface of said silica particles containing a fluorescent rare-earth complex of the present invention, and therefore, the present invention provides silica particles containing a fluorescent rare-earth complex, introduced with the above-described bonding groups or intercalator molecules at the surface of silica particles of the present invention.

Furthermore, background noise caused by non-specific adsorption can be reduced by using monovalent intercalator molecules having only one reactive moiety, comparing with bivalent intercalator molecules having two reactive moieties with particles. As these intercalator molecules, an intercalator molecule represented by the following formula is included:

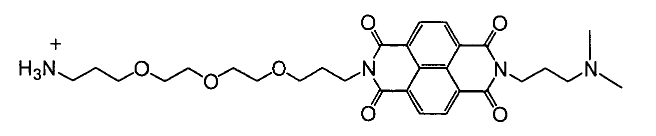

The present invention enables to bond silica particles containing a fluorescent rare-earth complex of the present invention to various molecular species specifically bondable, directly or through the above-described bonding groups, and thus can be used as a fluorescent labeling agent for these molecular species. Therefore, the present invention provides a fluorescent labeling agent consisting of the above-described silica particles containing a fluorescent rare-earth complex of the present invention.

A fluorescent labeling agent of the present invention can be used similarly as a usual fluorescent labeling agent, however, because surface of the silica particles is substantially silica, it also fulfills function as a carrier for immobilization and also has fluidity as particles.

Furthermore, a fluorescent labeling agent comprising silica particles containing a fluorescent rare-earth complex of the present invention can be used as a marker, in more detail, as a fluorescent maker by bonding to molecular species specifically bondable, such as an antibody, an antigen, a receptor and a nucleic acid probe.

In addition, by using molecular species containing a fluorescent labeling agent of the present invention, or a marker containing said fluorescent labeling agent, target molecules can be determined. As such a determination method, a usual fluorescent determination method, or a time-resolved fluorometry utilizing characteristics of a fluorescent rare-earth complex is included. In addition, a kit for target molecule determination can be obtained by combination of material for target molecule determination such as a buffer solution or a container, required in determination of a target molecule using molecular species containing a fluorescent labeling agent of the present invention, or a marker containing said fluorescent labeling agent. Therefore, the present invention provides a kit for target molecule determination, comprising molecular species containing a fluorescent labeling agent, or a marker containing said fluorescent labeling agent, and material for target molecule determination.

Content described in specification of Japanese Patent application No.2003-316006 is entirely incorporated into this specification.

### EXAMPLE

The present invention is described more specifically using Examples, however, the present invention is by no means limited to these Examples.

### Example 1

Synthesis of silica particles containing a terbium complex of N,N,N',N'-{2,6-bis(3'-aminomethyl-1'-pyrazolyl)-4-phenylpyridine}tetrakis(acetic acid) (abbreviated as BPTA)

7.5 ml of cyclohexane, 1.8 ml of n-hexanol and 3.54 ml of a surfactant (TritonX-100) were mixed and 340 µl of ion-exchanged water was added thereto, and subjected to ultrasonic irradiation to form emulsion. Thereto was added 50 µl of tetraethyl orthosilicate and a solution I was prepared. Into 280 µl of ion-exchanged water and 60 µl of ammonia water were dissolved 2.0 mg of BPTA and 1.5 mg of terbium chloride hexahydrates. This solution was added to a mixed solution of 7.5 ml of cyclohexane, 1.8 ml of n-hexanol and 3.54 ml of a surfactant (TritonX-100), and subjected to ultrasonic irradiation to form emulsion, which was named a solution II. The solution II was added dropwise to the solution I while stirring, and subsequently further stirred at room temperature for 20 hours. Acetone was added to the reaction mixture, and particles were obtained by centrifugation. These particles were washed with ethanol and water three times respectively to completely remove residual surfactant and a complex adsorbed at the surface. By drying under reduced pressure, 10 mg of white particles were obtained.

### Example 2

Synthesis of silica particles containing a europium complex of 2,2',2",2"'-[4'-biphenyl-2,2':6',2"-terpyridine-6,6"-diyl]bis(methylenenitrilo) tetrakis (acetic acid) (abbreviated as BTTA)

7.5 ml of cyclohexane, 1.8 ml of n-hexanol and 1.34 ml of a surfactant (Brij-97) were mixed and 1.36 ml of ion-exchanged water was added thereto, and subjected to ultrasonic irradiation to form emulsion. Thereto was added 100 µl of tetraethyl orthosilicate and a solution I was prepared. Into 1.24 ml of ion-exchanged water and 120 µl of a 2M aqueous solution of sodium hydroxide were dissolved 12 mg of BTTA and 8.0 mg of terbium chloride hexahydrates. This solution was added to a mixed solution of 7.5 ml of cyclohexane, 1.8 ml of n-hexanol and 1.34 ml of a surfactant (Brij-97), and subjected to ultrasonic irradiation to form emulsion, which was named a solution II. The solution I was stirred and the solution II was added dropwise thereto, and subsequently further stirred at room temperature for 20 hours. Acetone was added to the reaction mixture, and particles were obtained by centrifugation. These particles were washed with ethanol and water three times respectively to completely remove residual surfactant and a complex adsorbed at the surface. By drying under reduced pressure, 10 mg of white particles were obtained.

### Example 3

Silica particles obtained in Example 1 were dispersed in a 15 mM Tris-hydrochloric acid buffer solution (pH 7.4), a buffer solution of 15 mM Tris-hydrochloric acid and 50 mM sodium chloride (pH 7.4), a 1×SSC buffer solution, a 1×PBS buffer solution, a 1×TE buffer solution (pH 8.0) and a 100 mM carbonic acid buffer solution (pH 9.0) to prepare 1.0 mg/l of respective dispersion solution. These solutions were added to each well of a microtiter plate by 100 µl to be subjected to fluorescent determination.

As a blank experiment, BPTA-Tb³⁺ was added to a 15 mM Tris-hydrochloric acid buffer solution (pH 7.4), a buffer solution of 15 mM Tris-hydrochloric acid and 50 mM sodium chloride (pH 7.4), a 1×SSC buffer solution, a 1×PBS buffer solution, a 1×TE buffer solution (pH 8.0) and a 100 mM carbonic acid buffer solution (pH 9.0) so as to make 10⁻⁸ M, and these solutions were added to each well of the microtiter plate by 100 µl to be subjected to fluorescent determination.

Time-resolved fluorometry apparatus used in the present determination was ARVO-SX 1420 time-resolved fluorometry apparatus (produced from Wallac Co., Ltd.), and determination conditions were as follows: Excitation wavelength=320 nm, determination wavelength=545 nm, delay time=0.5 msec., window time=1.4 msec. and cycling time=2.0 msec.

Fluorescence intensity obtained by the above determination is shown in Fig. 2. The left side of Fig. 2 represents the blank experiment result, while the right side represents the case for silica particles of the present invention. In the blank example, in particular, in a 1×PBS buffer solution, a 1×TE buffer solution and a 100 mM carbonic acid buffer solution (pH 9.1), decrease in fluorescence intensity was observed. On the other hand, fluorescence for silica particles showed nearly constant value, which suggested that a fluorescent rare-earth complex was present inside the particles whereby protected from the effects of the surrounding environment such as a buffer solution.

### Example 4 Evaluation of fluorescence characteristics of silica particles

By dispersing silica particles obtained in Examples 1 and 2 into ion-exchanged water, 0.1 mg/ml of dispersion solutions were prepared. Fluorescence spectra using these solutions are shown each in Fig. 1 and Fig. 3. Spectra obtained were nearly the same as spectra of fluorescent rare-earth complexes introduced, and thus it was found that features of fluorescent rare-earth complexes in a solution were maintained although incorporated into particles.

### Example 5 Introduction of a cyano group to the surface of silica particles

Silica particles obtained in Examples 1 and 2 were dispersed in 9.0 ml of a 2 M aqueous solution of sodium carbonate, and 0.5 ml of an acetonitrile solution dissolved with 1.0 g of cyanogen bromide was added thereto, and stirred at room temperature for 10 minutes. After centrifugation of particles and subsequently washed with cold water and a PBS buffer solution twice respectively, white particles were obtained.

### Example 6 Introduction of intercalators to the surface of silica particles

Cyanized silica particles obtained in Example 5 were dispersed in 1 ml of a PBS buffer solution, and 10 ml of a 10 M PBS solution of intercalator (N,N'-bis(3-{2-[2-(3-aminopropoxy)-ethoxy]ethoxy}propyl)-1,4,5,8-tetracarboxydiimidenaphthalene trifluoroacetate salt) was added thereto, and was laid still at room temperature for 15 hours. Then, the reaction solution was removed by centrifugation, and 1.0 ml of 0.1 M PBS solution of glycine was added and stirred at room temperature for 1 hour. After centrifugation of particles and subsequently washed with a PBS buffer solution twice respectively, pale brown particles were obtained.

### Example 7 Labeling of DNA using silica particles relevant to the present invention

Using silica particles obtained in Example 6, a labeling experiment of DNA immobilized on the microtiter plate having 96 wells was carried out. A specific operation method is as follows:
(i) Immobilization of DNA: Double-stranded DNA (salmon testes) was dissolved in a buffer solution for immobilization (a 20 mM Tris-hydrochloric acid buffer solution, 150 mM magnesium chloride and 150 mM sodium chloride) to prepare 1 mg/ml of a DNA solution. This DNA solution was added to each well of the microtiter plate by 100 µl, and was laid still at room temperature overnight. After washing once with the buffer solution for immobilization, UV irradiation (254 nm, 1600 mJ/cm²) was carried out to secure immobilization.
(ii) Blocking of the microtiter plate: A blocking solution (a 100 mM Tris-hydrochloric acid buffer solution (pH 7.8), 100 mM sodium chloride, 2% bovine serum albumin (BSA) and 1% sodium azide) was added by 100 ml to each well of the microtiter plate, and placed at room temperature for 1 hour under light shielding and washed with a 0.01 × SSC buffer solution three times.
(iii) Labeling of DNA: 1 mg of silica particles containing a terbium complex obtained in Example 4 were dispersed in 600 µl of a 1×SSC buffer solution, and this solution was added by 100 µl to each well of the microtiter plate, placed at room temperature for 2 hours under light shielding, and washed with a 1×SSC buffer solution seven times to be subjected to fluorescent determination.

Time-resolved fluorometry apparatus used in the present determination was ARVO-SX 1420 time-resolved fluorometry apparatus (produced from Wallac Co., Ltd.) and determination conditions were as follows: Excitation wavelength=320 nm, determination wavelength=545 nm, delay time=0.5 msec., window time=1.4 msec. and cycling time=2.0 msec.

Fluorescence intensity obtained by the above determination and fluorescence intensity for the case without presence of DNA as a blank are shown in Fig. 4.

### Example 8

Configuration of particles obtained in Example 2 was observed by scanning electron microscope. Particle size was about 80 nm. Image obtained is shown in Fig. 5.

### Example 9 Introduction of intercalators to the surface of silica particles (2)

Cyanized silica particles obtained in Example 5 were dispersed in 5 ml of a 0.1 M carbonic acid buffer solution, and 250 ml of a carbonic acid buffer solution of 0.1 M intercalator (N-(3-{2-[2-(3-aminopropoxy)ethoxy]ethoxy}propyl)-N'-(N,N-dimethylaminopropyl)-1,4,5,8-te racarboxydiimidenaphthalene trifluoroacetate salt) was added thereto, and was laid still at room temperature for 15 hours. Then, the reaction solution was removed by centrifugation, and 1.0 ml of 0.1 M PBS solution of glycine was added, and stirred at room temperature for 1 hour. After centrifugation of particles, and subsequently washed with a PBS buffer solution twice, pale yellow particles were obtained.

### Example 10 Labeling of DNA using silica particles relevant to the present invention (1)

Using silica particles obtained in Example 1, DNA immobilized on the microtiter plate having 96 wells was labeled. A specific operation method is as follows:
(i) Immobilization of DNA: Double-stranded DNA (salmon testes) was dissolved in a buffer solution for immobilization (a 20 mM Tris-hydrochloric acid buffer solution, 150 mM magnesium chloride and 150 mM sodium chloride) to prepare 250 µg/ml of a DNA solution. This DNA solution was added to each well of the microtiter plate by 100 µl, and was laid still at room temperature overnight. After washing once with the buffer solution for immobilization, UV irradiation (254 nm, 1600 mJ/cm²) was carried out to secure immobilization. A sample of a single-stranded DNA was obtained by dissolving the above-described double-stranded DNA into the buffer solution for immobilization, heating at 95°C for 10 minutes, and quenched for thermal denaturation, and immobilized similarly as in the double-stranded DNA.
(ii) Blocking of the microtiter plate: A blocking solution (a 100 mM Tris-hydrochloric acid buffer solution (pH 7.8), 100 mM sodium chloride, 2% bovine serum albumin (BSA) and 1% sodium azide) was added by 100 ml to each well of the microtiter plate, and placed at room temperature for 1 hour under light shielding and washed with a 0.01×SSC buffer solution three times.
(iii) Alkali denaturation: As for a single-stranded DNA, after blocking operation, a solution of 0.1N NaoH and 0.1 M NaCl was added to each well by 100 ml, was laid still at room temperature for 20 minutes and washed three times with a 1×SSC buffer solution cooled at 4°C.
(iv) Labeling of DNA: 1 Mg of silica particles containing a terbium complex obtained in Example 6 were dispersed in 600 ml of a 1×SSC buffer solution, and this solution was added by 100 ml to each well of the microtiter plate, and placed at room temperature for 5 minutes under light shielding, washed with a 1×SSC buffer solution seven times to be subjected to fluorescent determination.

Time-resolved fluorometry apparatus used in the present determination was ARVO-SX 1420 time-resolved fluorometry apparatus (produced from Wallac Co., Ltd.) and determination conditions were as follows: Excitation wavelength=320 nm, determination wavelength=545 nm, delay time=0.5 msec., window time=1.4 msec. and cycling time=2.0 msec.

Fluorescence intensity obtained in the above determination is shown in Fig. 6.

### Example 11 Labeling of DNA using silica particles relevant to the present invention (2)

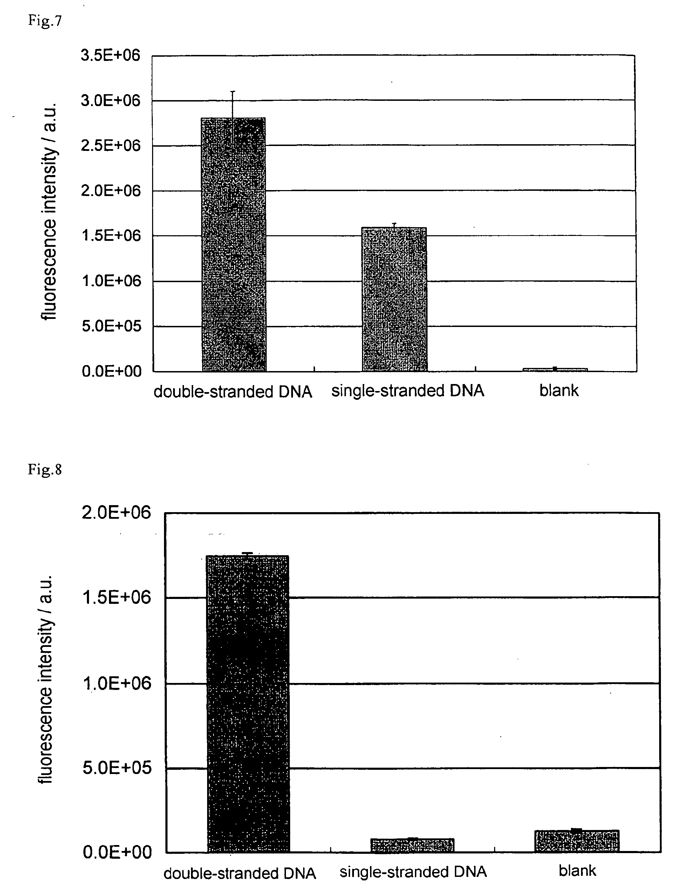

Using silica particles obtained in Example 6, DNA immobilized on the microtiter plate having 96 wells was labeled. A specific operation method is similar as in Example 10. Fluorescence intensity obtained by the determination and fluorescence intensity for the case without presence of DNA as a blank are shown in Fig. 7.

### Example 12 Labeling of DNA using silica particles relevant to the present invention (3)

Using silica particles obtained in Example 9, DNA immobilized on the microtiter plate having 96 wells was labeled. A specific operation method is as follows:
(i) Immobilization of DNA: Double-stranded DNA (M13mp18 RF DNA from TAKARA BIO INC.) or single-stranded DNA (M13mp18 RF Single Strand DNA produced from TAKARA BIO INC.) was dissolved in a buffer solution for immobilization (a 20 mM Tris-hydrochloric acid buffer solution, 150 mM magnesium chloride and 150 mM sodium chloride) to prepare 10 µg/ml of a DNA solution. This DNA solution was added to each well of the microtiter plate by 100 µl and stood still at 37°C overnight. After washing once with the buffer solution for immobilization, UV irradiation (254 nm, 1600 mJ/cm²) was carried out to secure immobilization.
(ii) Labeling of DNA: 1 Mg of silica particles containing a europium complex obtained in Example 9 were added in 2.5 ml of a 1×SSC buffer solution and dispersed by ultrasonic irradiation for 5 minutes. This solution was added to each well of the microtiter plate by 100 µl and placed at room temperature for 10 minutes under light shielding, washed with a 1×SSC buffer solution nine times and with an aqueous solution of 0.2% TritonX-100 nine times, to be subjected to fluorescent determination.

Time-resolved fluorometry apparatus used in the present determination was ARVO-SX 1420 time-resolved fluorometry apparatus (from Wallac Co., Ltd.) and determination conditions were as follows: Excitation wavelength=340 nm, determination wavelength=615 nm, delay time=0.1 msec., window time=1.5 msec. and cycling time=2.0 msec.

Fluorescence intensity obtained in the above determination and fluorescence intensity for the case without the presence of DNA as a blank are shown in Fig. 8.

### INDUSTRIAL APPLICABILITY

The present invention provides a labeling reagent having bonding groups to a substance to be labeled (for example, a bio-derived substance, a biologically active substance, and the like) at the surface, and comprising silica particles containing a fluorescent rare-earth complex inside. Because a complex inside said particles are uninfluenced by the surrounding environment and bonding groups to a substance to be labeled can be introduced at the silica surface, even a fluorescent rare-earth complex which conventionally could not be used as a labeling agent by causes such as difficulty in introduction of bonding groups, extreme decrease in fluorescence intensity by introduction of bonding groups, dissociation of a rare-earth metal due to insufficient chelating force, and the like, can be utilized as a labeling agent. Furthermore, because the particles contain many fluorescent complexes, fluorescence intensity is stronger compared with the case of direct labeling of a complex.

In addition, a fluorescent labeling agent introduced with intercalators relevant to the present invention enables to specifically label double-stranded DNA and thus can be directly applicable to DNA chips and the like.

The present invention provides silica particles containing a fluorescent rare-earth complex for fluorescent labeling wherein fluorescent label is contained inside silica particles. A fluorescent labeling agent containing silica particles of the present invention can be used as a fluorescent labeling agent for various fluorescent determinations, and not only usable as a conventional fluorescent labeling agent but also for a carrier for silica particles, and furthermore, because fluorescent substances are contained inside silica particles, fluorescent substances for labeling can stably exist, uninfluenced by the surrounding environment.

Therefore, silica particles of the present invention and a fluorescent labeling agent using thereof and a determination method using thereof are useful as various labeling agents for fluorescent determination, and thus have industrial applicability.

## Claims

1. Silica particles containing a fluorescent rare-earth complex comprising a fluorescent rare-earth complex.

2. The silica particles according to claim 1, wherein the fluorescent rare-earth complex comprises at least one kind of a compound selected from the group consisting of the compounds represented by the following formulae (1) to (6) 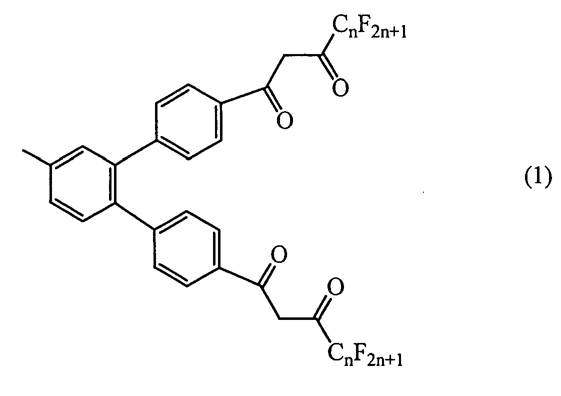 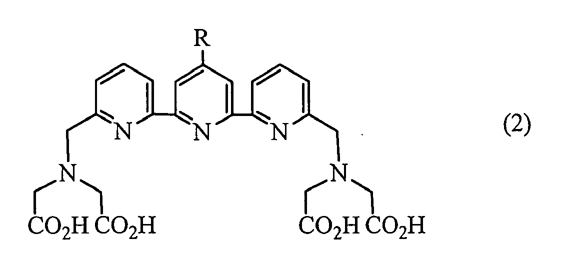 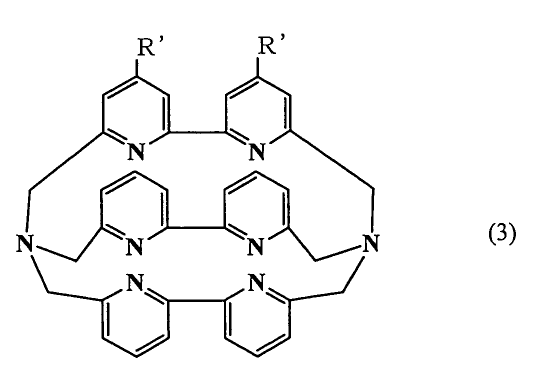 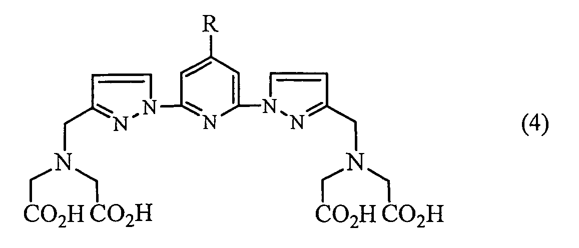 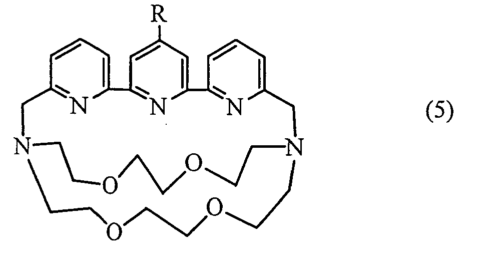 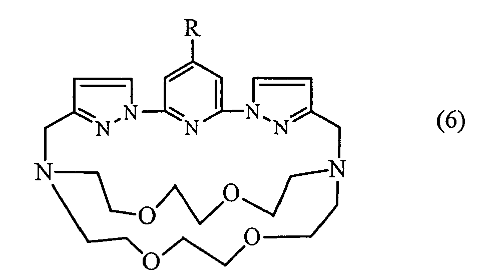 as a ligand.

3. The silica particles according to claim 1, wherein the fluorescent rare-earth complex comprising at least one kind of a compound selected from the group consisting of the compounds represented by the following formulae 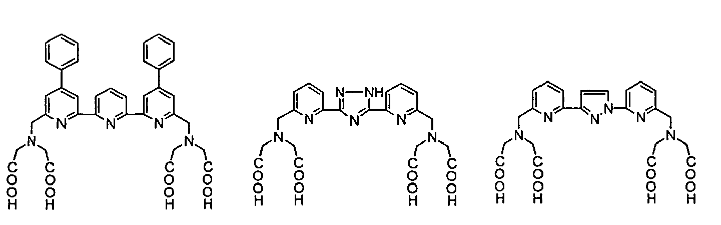 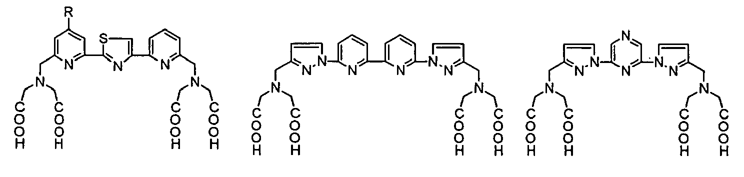 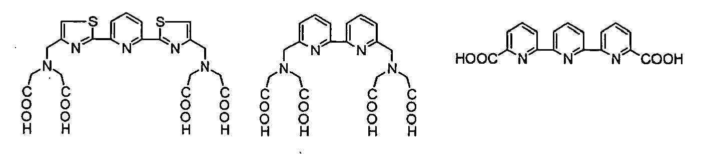 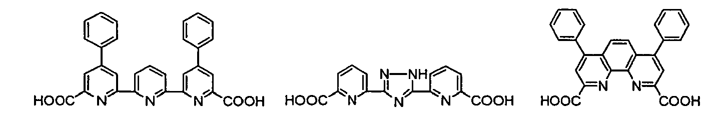 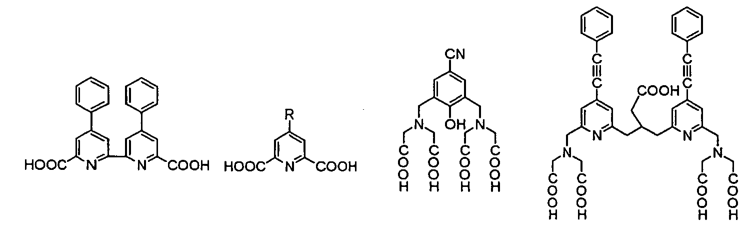 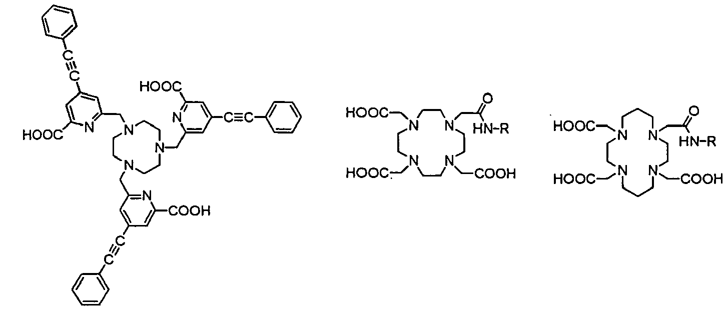 as a ligand.

4. The silica particles according to claim 1, wherein the fluorescent rare-earth complex comprising at least one kind of a compound selected from the group consisting of the compounds represented by the following formulae 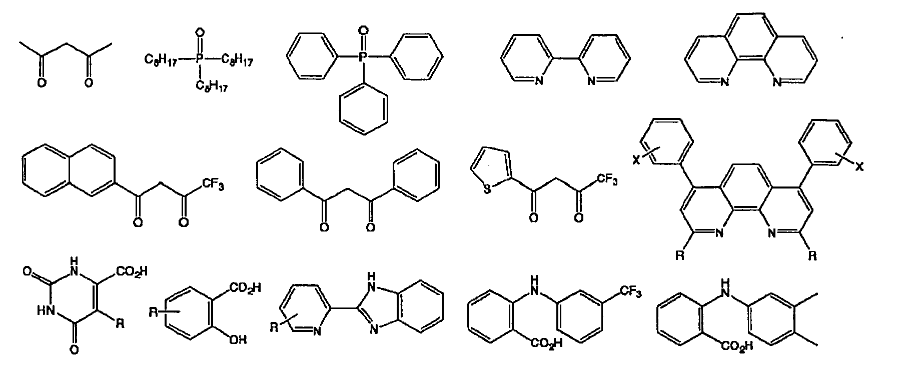 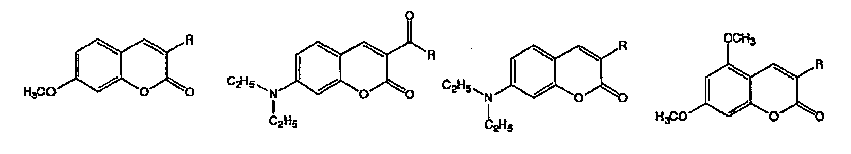 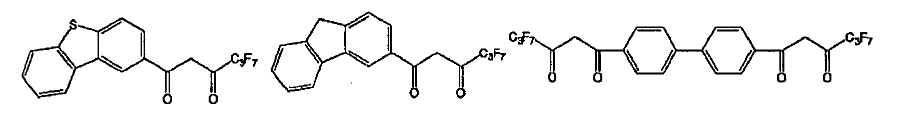 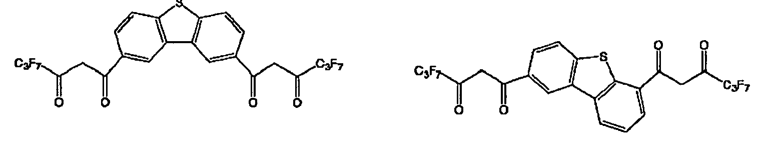 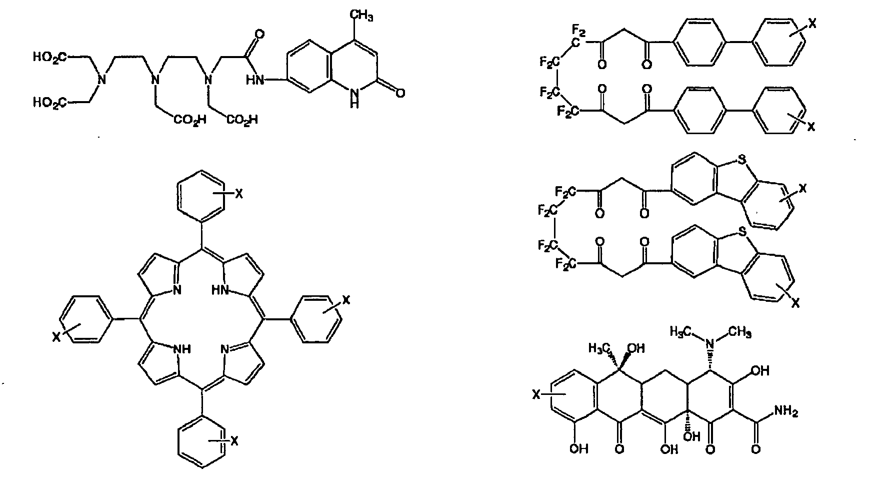 as a ligand.

5. The silica particles according to any one of claims 1 to 4, wherein the fluorescent rare-earth complex is a complex of terbium or europium.

6. The silica particles according to any one of claims 1 to 5, wherein the silica particles containing a fluorescent rare-earth complex is produced by an emulsion polymerization method.

7. The silica particles according to any one of claims 1 to 6, wherein silica particles selectively contain the fluorescent rare-earth complex mainly in an inner layer thereof.

8. The silica particles according to any one of claims 1 to 7, wherein the bonding groups to a substance to be labeled is introduced to the silica particles containing the fluorescent rare-earth complex.

9. The silica particles according to claim 8, wherein the bonding groups are cyano groups.

10. The silica particles according to any one of claims 1 to 9, wherein intercalator to double-stranded DNA is introduced to the silica particles containing the fluorescent rare-earth complex.

11. The silica particles according to any one of claims 1 to 9, wherein intercalator to double-stranded RNA is introduced to the silica particles containing the fluorescent rare-earth complex.

12. The silica particles according to any one of claims 1 to 9, wherein intercalator to double-stranded PNA is introduced to the silica particles containing the fluorescent rare-earth complex.

13. The silica particles according to any one of claims 1 to 9, wherein intercalator to double-stranded DNA/RNA hybrid is introduced to the silica particles containing the fluorescent rare-earth complex.

14. The silica particles according to any one of claims 1 to 9, wherein intercalator to double-stranded DNA/PNA hybrid is introduced to the silica particles containing the fluorescent rare-earth complex.

15. The silica particles according to any one of claims 1 to 9, wherein intercalator to double-stranded RNA/PNA hybrid is introduced to the silica particles containing the fluorescent rare-earth complex.

16. The silica particles according to any one of claims 10 to 15, wherein intercalator is monovalent.

17. A fluorescent labeling agent comprising the silica particles according to any one of claims 1 to 16.

18. A fluorescence labeled nucleic acid probe, wherein the nucleic acid probe is bonded at the surface of the silica particles according to any one of claims 1 to 16.

19. The nucleic acid probe according to claim 18, wherein the nucleic acid is DNA.

20. The nucleic acid probe according to claim 18, wherein the nucleic acid is RNA.

21. The nucleic acid probe according to claim 18, wherein the nucleic acid is PNA.

22. A method for detection of double-stranded DNA using the silica particles containing the fluorescent rare-earth complex, which is introduced with the intercalator to the double-stranded DNA according to claim 10.

23. A method for detection of double-stranded RNA using silica particles containing the fluorescent rare-earth complex, which is introduced with the intercalator to the double-stranded RNA according to claim 11.

24. A method for detection of double-stranded PNA using the silica particles containing the fluorescent rare-earth complex, which is introduced with the intercalator to the double-stranded PNA according to claim 12.

25. A method for detection of a double-stranded DNA/RNA hybrid using the silica particles containing the fluorescent rare-earth complex, which is introduced with the intercalator to the double-stranded DNA/RNA hybrid according to claim 13.

26. A method for detection of a double-stranded DNA/PNA hybrid using the silica particles containing the fluorescent rare-earth complex, which is introduced with the intercalator to the double-stranded DNA/PNA hybrid according to claim 14.

27. A method for detection of a double-stranded RNA/PNA hybrid using the silica particles containing the fluorescent rare-earth complex, which is introduced with the intercalator to the double-stranded RNA/PNA hybrid according to claim 15.

28. A kit for target molecule determination comprising a molecular species containing the fluorescent labeling agent according to claim 17, or markers containing said fluorescent labeling agent, and material for target molecule determination.
